(19) Europäisches Patentamt
European Patent Office
Office européen des brevets

(11) Numéro de publication: **0 081 699**
**B1**

(12)
# FASCICULE DE BREVET EUROPEEN

(45) Date de publication du fascicule du brevet: **23.01.85**

(21) Numéro de dépôt: **82110711.7**

(22) Date de dépôt: **19.11.82**

(51) Int. Cl.[4]: **C 07 C 43/196**, C 07 C 41/03, A 23 L 1/226, A 24 B 3/12, C 11 B 9/00

(54) **Nouveaux composés alicycliques, leur utilisation à titre d'ingrédients parfumants ou aromatisants, procédé pour leur préparation.**

(30) Priorité: **10.12.81 CH 7888/81**

(43) Date de publication de la demande: **22.06.83 Bulletin 83/25**

(45) Mention de la délivrance du brevet: **23.01.85 Bulletin 85/4**

(84) Etats contractants désignés: **CH DE FR GB LI NL**

(56) Documents cités:
**FR - A - 2 395 751**
**FR - A - 2 418 214**

(73) Titulaire: **FIRMENICH SA, 1, route des Jeunes, CH-1211 Genève 8 (CH)**

(72) Inventeur: **Giersch, Wolfgang Klaus, 353, Route de Bernex, CH-1233 Bernex (CH)**
Inventeur: **Schulte-Elte, Karl-Heinrich, Dr., 44, Chemin de Carabot, CH-1213 Onex (CH)**
Inventeur: **Ohloff, Günther, Dr., 13, Chemin de la Chapelle, CH-1233 Bernex (CH)**

(74) Mandataire: **Salvadori, Giuseppe, Dr., c/o Firmenich S.A. Case Postale 239, CH-1211 Genève 8 (CH)**

EP 0 081 699 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

## Description

L'invention traite du domaine des parfums et arômes. Elle a plus particulièrement pour objet des composés nouveaux de formule

$$X—CH_2—CH(OH)—CH_2—R \qquad (I)$$

dans laquelle le symbole R représente un radical alkyle contenant de 1 à 3 atomes de carbone et X un radical cyclohexyloxy substitué de formule

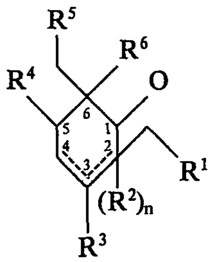

(II)

dans laquelle l'indice n vaut zéro ou 1, les pointillés désignent l'emplacement d'une simple (n = 1) ou double liaison en position 2 (n = 0) ou 3 (n = 1) et chacun des symboles $R^1$ à $R^6$ représente un atome d'hydrogène ou un radical méthyle.

Elle a également pour objet l'utilisation des composés (I) définis ci-dessus à titre d'ingrédients parfumants ou aromatisants, de même qu'une composiotion parfumante ou aromatisante contenant les composés (I) à titre d'ingrédients organoleptiquement actifs. Elle a en outre pour objet un procédé pour la préparation desdits composés (I).

L'invention est définie aux revendications.

A titre de composés (I) préférentiels, on peut citer les composés de formule (I) dans laquelle le symbole X représente un radical cyclohexyloxy substitué de formule

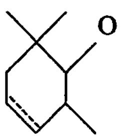
(IIa)

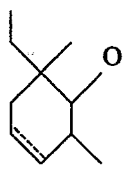
(IIb)

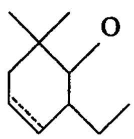
(IIc)

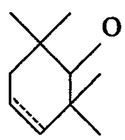
(IId)

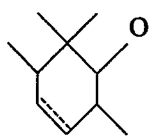
(IIe)

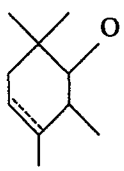
(IIf)

ou

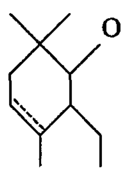
(IIg)

dans laquelle les pointillés désignent l'emplacement d'une simple ou double liaison.

Parmi les composés ci-dessus, on mentionnera plus particulièrement le 1-(2,6,6-triméthyl-cyclohex-1-yloxy)-pentane-2-ol, le 1-(2,6,6-triméthyl-cyclohex-3-ène-1-yloxy)-pentane-2-ol, 1-(2,3,6,6-tétraméthyl-cyclohex-1-yloxy)-pentane-2-ol, le 1-(2,5,6,6-tétraméthyl-cyclohex-1-yloxy)-pentane-2-ol, le 1-(2-éthyl-6,6-diméthyl-cyclohex-1-yloxy)-pentane-2-ol et le 1-(2,6,6-triméthyl-cyclohex-1-yloxy)-butane-2-ol.

L'une des préoccupations essentielles de l'industrie des parfums, tout comme celle des arômes, consiste à disposer d'un éventail toujours plus large d'ingrédients parfumants, respectivement aromatisants. Ce n'est qu'ainsi que l'on peut en effet créer des notes nouvelles ou procéder au remplacement de corps odorants devenus trop onéreux ou ayant disparu du marché. Par cet apport, l'industrie chimique contribue en outre à réduire la dépendance de la parfumerie, par exemple, vis à vis des produits d'origine naturelle.

L'une des classes de corps odorants les plus intensément exploitées est sans conteste celle des corps boisés: elle est en effet constituée de corps chimiques des plus variés tels des alcools, des esters, des cétones ou des éthers à structure monocyclique, macrocyclique ou encore bi-, tri- ou polycyclique par

exemple. Parmi les corps odorants boisés à structure alicyclique, on peut citer entre autres des éthers aliphatiques dérivant de composés iononiques, tels par exemple les composés de formules

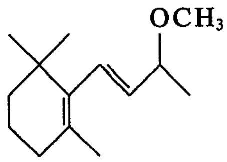 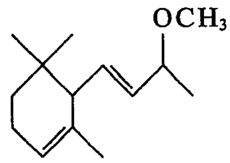 et 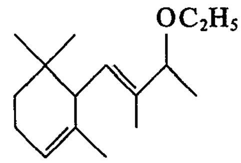

qui développent une odeur générique boisée accompagnée d'une tonalité fruitée, ambrée ou tabac selon les cas (voir à ce sujet DE-OS 2 287 636).

Dans ce même contexte, on peut également citer divers homologues méthylés de la $\beta$-ionone par exemple (voir à ce sujet S. Arctander, Perfume and Flavor Chemicals, Montclair N. J. 1969, sections nos. 2087 et 2088) et certains de leurs mélanges. L'alcool alicyclique de formule

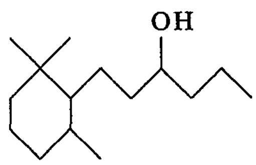

finalement, qui s'utilise comme fixateur pour parfums, développe selon certains, une odeur de type boisé-ambré (voir à ce sujet DE-OS 2 807 584).

Vu l'abondance de corps odorants boisés à structure iononique répertoriés dans l'art antérieur, il y avait tout lieu de penser que cette série particulière figurait dans sa totalité sur la palette du parfumeur. Contrairement à toute attente cependant, il a été trouvé que les composés alicycliques oxygénés de formule (I) tels que définis précédemment présentaient d'intéressantes propriétés organoleptiques et qu'ils pouvaient être de ce fait avantageusement utilisés dans l'industrie des parfums tout comme celle des arômes.

Dans le domaine des parfums, les composés de formule (I) se distinguent par leur odeur boisée-ambrée élégante, chaude et montante, enrichie d'une tonalité légèrement animale rappelant celle de l'ambre naturel, parfois faiblement fruitée ou fleurie selon les cas. Puissants mais de ténacité moyenne, ils conviennent particulièrement bien à la préparation de compositions parfumantes fort variées, telles des compositions de type chypre, rosé, boisé ou fougère par exemple, compositions auxquelles ils confèrent harmonie et richesse, sans que la tonalité boisée-ambrée qui les caractérise ne devienne exagérément dominante. Les composés de formule (I) peuvent être en outre utilisés pour la préparation de produits parfumés tels que savons, détergents, produits d'entretien ou préparations cosmétiques, notamment de préparations cosmétiques de type masculin, eaux de toilette ou lotions après rasage par exemple.

Les quantités à utiliser pour obtenir un effet parfumant tel que défini ci-dessus pourront varier de façon étendue, en fonction notamment de la nature des coingrédients d'une composition donnée ou de la nature des produits auxquels on désire les ajouter. Pour la préparation de compositions parfumantes par exemple, on peut avantageusement utiliser les composés de formule (I) dans des proportions comprises entre environ 1 et 15% (poids), parfois 30% ou plus lors de la préparation de bases ou cœurs pour parfums par exemple. Les quantités ci-dessus ne sont cependant indiquées qu'à titre indicatif.

Du point de vue gustatif, les composés de formule (I) se caractérisent par un goût et un arôme que l'on peut qualifier de tout à la fois boisé et ambre. Ils peuvent être de ce fait avantageusement utilisés pour la préparation d'arômes artificiels variés plus particulièrement destinés à l'aromatisation du tabac ou de produits à base de tabac. Lesdits composés sont notamment appréciés à titre d'ingrédients aromatisants lors de la préparation de mélanges de tabacs pour la pipe ou pour les cigarettes par exemple, mélanges auxquels ils confèrent une note tout à la fois boisée et ambrée rappelant le goût et l'arôme d'un mélange de tabacs orientaux.

La nature, la solubilité et la stabilité des composés de formule (I) déterminent le plus souvent la forme sous laquelle ils doivent être utilisés pour l'aromatisation d'un tabac. Lesdits composés peuvent être incorporés à tout moment, lors du traitement du tabac ou mélange de tabacs en question, de préférence après les phases de vieillissement, séchage et coupage des feuilles de tabac, avant par exemple la manufacture des cigarettes.

L'aromatisation du tabac s'effectue généralement par aspersion du mélange considéré au moyen d'une solution du composé de formule (I) choisi dans l'éthanol, ou parfois dans un mélange d'éthanol et de propylène-glycol.

Les composés de formule (I) peuvent être utilisés aux fins décrites ci-dessus dans des proportions variant de façon étendue. Lors de l'aromatisation du tabac ou de produits à base de tabac, on peut les utiliser à raison de 1 à 100 ppm par rapport au poids de la matière à traiter, de préférence à raison de 5 à

10 ppm. Il demeure entendu cependant que des quantités supérieures ou inférieures aux limites données ci-dessus peuvent être également utilisées, notamment lorsque des effets plus particuliers sont recherchés.

Selon l'invention, les composés de formule (I) s'obtiennent à partir d'alcools alicycliques de formule

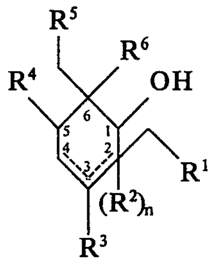

(IV)

dans laquelles les symboles $R^1$ à $R^6$, l'indice n et les pointillés sont définis comme indiqué précédemment pour la formule (I), par réaction de ces derniers avec un composé de formule

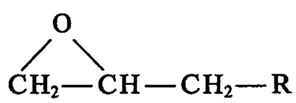

(III)

dans laquelle le symbole R représente un radical alkyle contenant de 1 à 3 atomes de carbone. Selon le procédé de l'invention, ladite réaction s'effectue en présence d'une base forte telle un hydrure de métal alcalin, l'hydrure de sodium ou lithium par exemple ou encore un métal alcalin, le sodium par exemple. Ladite réaction s'effectue en outre en présence d'un solvant organique inerte tel un éther ou un hydrocarbure aliphatique ou aromatique, éther de pétrole ou toluène par exemple (voir à ce sujet Houben-Weyl, Methoden der organischen Chemie, Georg Thieme Verlag, Stuttgart 1965, Vol. VI/3).

Les alcools alicycliques de formule (IV) utilisés à titre de produits de départ selon le prodédé de l'invention peuvent être obtenus soit directement sur le marché, auprès des fournisseurs usuels, soit après réduction des cétones alicycliques correspondantes selon les techniques conventionnelles. Les composés époxydés de formule (III) sont de produits du commerce.

Tels que directement issus de la synthèse, les composés de formule (I) peuvent se présenter sous diverses formes stéréoisomères, en fonction des diverses possibilités de substitution du cycle à six membres, formes que l'on peut selon les cas distinguer l'une de l'autre à l'aide de techniques de séparation et d'identification appropriées. Selon les cas, ces formes stéréoisomères peuvent présenter à côte de leur note boisée-ambrée commune d'autres nuances olfactives. Cependant, pour des raisons d'ordre pratique et économique, on utilisera de préférence des mélanges de stéréoisomères tels que provenant directement de la synthèse.

L'invention sera illustrée, de façon non limitative, par les exemples ci-dessous (températures en degrés centigrades).

### Exemple 1

1-(2,6,6-Triméthyl-cyclohex-1-yloxy)-pentane-2-ol

2,0 g de 2,6,6-triméthyl-cyclohexanol et 0,354 g d'hydrure de sodium ont été chauffés durant 24 h à 80° dans 10 ml de toluène absolu. 1,2 g de 1,2-époxypentane ont ensuite été ajoutés à la solution ainsi obtenue et le chauffage à 80° a été poursuivi durant 24 h. Après refroidissement, le mélange de réaction a été versé sur de la glace acidifiée avec $H_2SO_4$ dilué, puis la phase organique lavée avec de la saumure jusqu'à neutralité. Après évaporation des parties volatiles et distillation du résidu dans un tube à boules (température du bain: 140°/7 Pa), on a recueilli le produit désiré avec un rendement de 75% environ. Un échantillon pour analyse a été purifié par chromatographie en phase gazeuse préparative.

IR: 3450 $cm^{-1}$;
RMN: signaux à 1,0; 1,2–1,6; 2,5; 2,8; 3,4 et 3,75 $\delta$ ppm;
SM: $M^+$ = 228(68); m/e: 157(25), 142(20), 125(45), 109(29), 95(16), 82(69), 69(100), 55(38), 41(48).
Qualification olfactive: boisé-ambré; puissant, montant; légère note animale du genre ambre naturel.

## Exemple 2

Par l'application du procédé l'Exemple 1 aux composés mentionnés ci-après, on a préparé les dérivés suivants:

a) 1-(2,6,6-triméthyl-cyclohex-1-yloxy)-butane-2-ol

produits de départ: 2,6,6-triméthyl-cyclohexanol
1,2-époxy-butane
IR: 3430 $cm^{-1}$;
RMN: signaux à 1,0; 2,5; 2,8 et 3,4—4,0 $\delta$ ppm;
SM: $M^+$ = 214(99); m/e: 143(45), 125(53), 109(37), 95(22), 82(90), 69(100), 55(66); 41(51).
Qualification olfactive: boisé-ambré; puissant modérée.

b) 1-(2,6,6-triméthyl-cyclohex-2-ène-1-yloxy)-butane-2-ol

produits de départ: 2,6,6-triméthyl-cyclohex-2-ène-1-ol
1,2-époxy-butane
RMN: signaux à 0,88; 0,97; 0,98; 1,78; 2,5; 3,13; 3,4—3,75 et 5,43 $\delta$ ppm;
SM: $M^+$ = 212(1); m/e: 156(63), 123(35), 107(21), 84(100), 73(40), 57(39), 43(63).
Qualification olfactive: boisé, cuir; légère note ambrée-animale et safran.

c) 1-(2,6,6-triméthyl-cyclohex-2-ène-1-yloxy)-pentane-2-ol

produits de départ: 2,6,6-triméthyl-cyclohex-2-ène-1-ol
1,2-époxy-butane
RMN: signaux à 0,88; 0,93; 0,97; 1,79; 2,45; 3,13; 3,4—4,0 et 5,46 $\delta$ ppm;
SM: m/e: 170(52), 123(28), 107(12), 84(100), 69(11), 55(16), 41(23).
Qualification olfactive: boisé, faiblement fruité; légèrement moisi.

d) 1-(2,6,6-triméthyl-cyclohex-3-ène-1-yloxy)-pentane-2-ol

produits de départ: 2,6,6-triméthyl-cyclohex-3-ène-1-ol
1,2-époxy-pentane
RMN: signaux à 1,0; 2,5; 2,92; 3,3—4,0 et 5,2—5,6 $\delta$ ppm;
SM: m/e: 158(72), 123(13), 107(8), 97(4), 87(16), 72(100), 55(14), 41(22).
Qualification olfactive: boisé-ambré typique; puissant.

e) 1-(2,2,6,6-tétramethyl-cyclohex-1-yloxy)-butane-2-ol

produits de départ: 2,2,6,6-tétraméthyl-cyclohexanol
1,2-époxy-butane
IR: 3440 $cm^{-1}$;
RMN: signaux à 1,0; 2,67 et 3,45—3,9 $\delta$ ppm;
SM: $M^+$ = 228(1); m/e: 138(21), 109(24), 82(100), 69(28), 55(22), 41(20).

f) 1-(2,2,6,6-tétraméthyl-cyclohex-1-yloxy)-pentane-2-ol

produits de départ: 2,2,6,6-tétraméthyl-cyclohexanol
1,2-époxy-pentane
IR: 3460 $cm^{-1}$;
RMN: signaux à 0,95; 2,67 et 3,4—4,0 $\delta$ ppm;
SM: $M^+$ = 242(1); m/e: 138(21), 109(22), 82(100), 69(31), 55(14), 41(21).

g) 1-(2,2,6,6-tétraméthyl-cyclohex-1-yloxy)-hexane-2-ol

produits de départ: 2,2,6,6-tétraméthyl-cyclohexanol
1,2-époxy-hexane
IR: 3450 $cm^{-1}$;
RMN: signaux à 0,95; 2,38 et 3,4—4,0 $\delta$ ppm;
SM: $M^+$ = 256(1); m/e: 138(20), 109(24), 82(100), 69(30), 55(25), 41(31).

h) 1-(2,5,6,6-tétraméthyl-cyclohex-1-yloxy)-pentane-2-ol

produits de départ: 2,5,6,6-tétraméthyl-cyclohexanol
1,2-époxy-pentane
IR: 3450 $cm^{-1}$;
RMN: signaux à 0,9; 2,41 et 3,1—4,0 $\delta$ ppm;
SM: $M^+$ = 242(42); m/e: 157(22), 138(99), 123(40), 109(22), 96(54), 83(95), 69(100), 55(90), 41(93).
Qualification olfactive: boisé-ambré caractéristique; note animale du genre ambre naturel; puissant et tenace.

i) 1-(2-éthyl-6,6-diméthyl-cyclohex-1-yloxy)-pentane-2-ol

produits de départ: 2-éthyl-6,6-diméthyl-cyclohexanol
1,2-époxy-pentane
IR: 3450 $cm^{-1}$;
RMN: signaux à 0,9; 2,53 et 3,2—4,0 $\delta$ ppm;
SM: $M^+$ = 242(67); m/e: 171(22), 139(22), 109(35), 82(100), 69(78), 55(62), 41(79).
Qualification olfactive: boisé-ambré; moins puissant que h).

j) 1-(t-2,t-6-diméthyl-r-1-cyclohexyloxy)-pentane-2-ol

produits de départ: t-2,t-6-diméthyl-cyclohexanol
1,2-époxy-pentane
RMN (90 MHz): 0,99; 2,5; 3,25—3,95 $\delta$ ppm;
SM: $M^+$ = 214(27); m/e: 111(100), 71(57), 69(97), 55(58), 41(40).
Qualification olfactive: légèrement boisé.

k) 1-(c-2,t-6-diméthyl-r-1-cyclohexyloxy)-pentane-2-ol

produits de départ: c-2,t-6-diméthyl-cyclohexanol
1,2-époxy-pentane
RMN (90 MHz): 0,90 et 0,94; 2,8—3,9 $\delta$ ppm;
SM: $M^+$ = 214(22); m/e: 111(100), 71(38), 69(83), 55(44), 41(29).
Qualification olfactive: boisé, transpiration.

## Exemple 3

On a préparé une composition parfumante de base pour eau de toilette masculine à partir des ingrédients ci-après:

| Ingrédients | Parties en poids |
|---|---|
| Essence de bergamote synth. | 150 |
| Acétate de cédryle | 150 |
| Galbanum résinoïde | 100 |
| Essence de citron d'Italie | 80 |
| Absolu de mousse de chêne décoloré à 10%*) | 80 |
| Essence de clous de girofle | 60 |
| EXALTEX®1) | 40 |
| Musc cétone | 40 |
| Essence de lavande | 40 |
| Essence de racines d'angélique à 10%*) | 40 |

Suite

| Ingrédients | Parties en poids |
|---|---|
| Essence de sauge sclarée | 30 |
| Néroli bigarade | 20 |
| Essence d'orange de Guinée | 20 |
| Essence de basilic à 10%*) | 20 |
| Absolu de jasmin synth. | 10 |
| FIXATEUR 404[1]) à 10%*) | 10 |
| Essence de labdanum ciste à 10%*) | 10 |
| Essence de thym rouge à 10%*) | 10 |
| Essence d'armoise | 10 |
| Total | 920 |

*) dans le phtalate diéthylique
[1]) origine: FIRMENICH SA — Genève/Suisse

La base parfumante ainsi préparée peut être qualifiée de type boisé-hespéridé. Lorsqu'on ajoute à 920 g de ladite base 80 g de 1-(2,6,6-triméthyl-cyclohex-1-yloxy)-pentane-2-ol, on obtient une composition parfumante dont l'odeur est devenue plus riche et plus arrondie et dont la tonalité boisée se démarque avec plus d'élégance.

En remplaçant dans l'Exemple ci-dessus l'ingrédient parfumant sus-mentionné par certains des composés cités à l'Exemple 2, on a observé ce qui suit (proportions identiques):

- 1-(2,6,6-triméthyl-cyclohex-1-yloxy)-butane-2-ol: effet analogue, un peu moins marqué
- 1-(2,6,6-triméthyl-cyclohex-3-ène-1-yloxy)-pentane-2-ol: bon effet boisé, riche
- 1-(2,5,6,6-tétraméthyl-cyclohex-1-yloxy)-pentane-2-ol: effet boisé très élégant, encore mieux marqué
- 1-(2-éthyl-6,6-diméthyl-cyclohex-1-yloxy)-pentane-2-ol: bon effet boisé, un peu moins net.

Exemple 4

10 g d'une solution de 1-(2,6,6-triméthyl-cyclohex-1-yloxy)-pentane-2-ol à 0,01 % dans l'éthanol à 95 % ont été vaporisés sur 100 g d'un mélange de tabacs de type »american blend«. Le tabac ainsi traité a ensuite été utilisé pour la préparation de cigarettes »test« dont la fumée a été soumises à l'évaluation organoleptique d'un groupe de personnes expertes. Après avoir comparé la fumée de ces cigarettes à celle de cigarettes dont le tabac n'avait été traité qu'au moyen d'éthanol à 95 % (cigarettes »témoin«), celles-ci se sont trouvées unanimes à déclarer que la fumée des cigarettes »test« présentait une note boisée et ambrée plus soutenue que celle de la fumée des cigarettes »témoin«, rappelant le goût et l'arôme de la fumée de cigarettes de type oriental.

En remplaçant dans l'exemple ci-dessus le 1-(2,6,6-triméthyl-cyclohex-1-yloxy)-pentane-2-ol par le 1-(2,5,6,6-tétraméthyl-cyclohex-1-yloxy)-pentane-2-ol en proportions identiques, on a observé un effet analogue.

**Revendications**

1. Composés de formule

$$X—CH_2—CH(OH)—CH_2—R \qquad (I)$$

dans laquelle le symbole R représente un radical alkyle contenant de 1 à 3 atomes de carbone et X un radical cyclohexyloxy substitué de formule

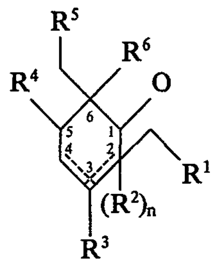

(II)

dans laquelle l'indice n vaut zéro ou 1, les pointillés désignent l'emplacement d'une simple (n = 1) ou double liaison en position 2 (n = 0) ou 3 (n = 1) et chacun des symboles $R^1$ à $R^6$ représente un atome d'hydrogène ou un radical méthyle.

2. A titre de composés selon la revendication 1, les composés de formule (I) dans laquelle le symbole X représente un radical cyclohexyloxy substitué de formule

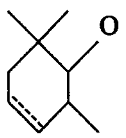 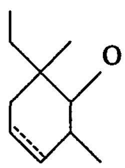 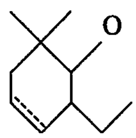 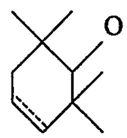

(IIa) (IIb) (IIc) (IId)

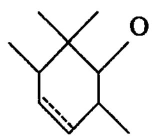 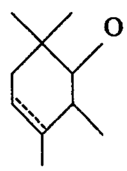 ou 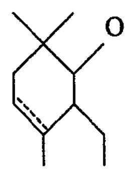

(IIe) (IIf) (IIg)

dans laquelle les pointillés désignent l'emplacement d'une simple ou double liaison.

3. A titre de composés selon l'une des revendications 1 et 2, l'un des composés mentionnés ci-après:

1-(2,6,6-triméthyl-cyclohex-1-yloxy)-pentane-2-ol,
1-(2,6,6-triméthyl-cyclohex-3-ène-1-yloxy)-pentane-2-ol,
1-(2,5,6,6-tétraméthyl-cyclohex-1-yloxy)-pentane-2-ol,
1-(2-éthyl-6,6-diméthyl-cyclohex-1-yloxy)-pentane-2-ol et
1-(2,6,6-triméthyl-cyclohex-1-yloxy)-butane-2-ol.

4. Procédé pour la préparation d'un composé selon la revendication 1, caractérisé en ce qu'on fait réagir en présence d'une base forte un composé de formule

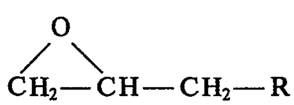

(III)

dans laquelle le symbole R représente un radical alkyle contenant de 1 à 3 atomes de carbone, avec un composé de formule

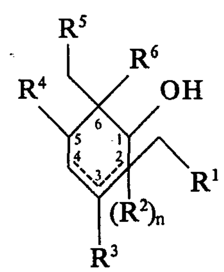

(IV)

dans laquelles les symboles $R^1$ à $R^6$, l'indice n et les pointillés sont définis comme pour la formule (I).

5. Utilisation à titre d'ingrédient parfumant ou aromatisant d'un composé selon l'une des revendications 1 à 3.

6. Composition parfumante ou aromatisante, caractérisée en ce qu'elle contient à titre d'ingrédient organoleptiquement actif un composé selon l'une des revendications 1 à 3.

7. Un produit parfumé, caractérisé en ce qu'il contient à titre d'ingrédient odoriférant un composé selon l'une des revendications 1 à 3.

8. Un aliment, une boisson, une préparation pharmaceutique ou un tabac, caractérisés en ce qu'ils contiennent à titre d'ingrédient aromatisant un composé selon l'une des revendications 1 à 3.

**Patentansprüche**

1. Verbindungen der Formel

$$X—CH_2—CH(OH)—CH_2—R \qquad (I)$$

worin das Symbol R einen C-1 bis C-3 Alkylrest und X einen substituierten Cyclohexyloxyrest der Formel

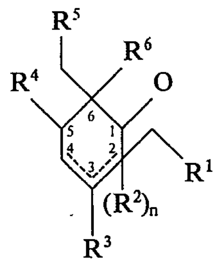

(II)

bedeuten, worin der Index n für Null oder 1 steht, die gestrichelten Linien eine Einfachbindung (n = 1) oder eine Doppelbindung in Stellung 2 (n = 0) oder 3 (n = 1) bezeichnen, und jedes der Symbole $R^1$ bis $R^6$ ein Wasserstoffatom oder einen Methylrest bedeutet.

2. Verbindungen der Formel (I) gemäß Anspruch 1, worin das Symbol X einen substituierten Cyclohexyloxyrest der Formel

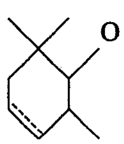

(IIa)

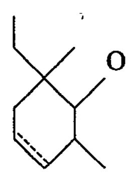

(IIb)

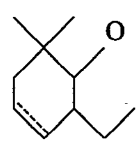

(IIc)

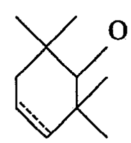

(IId)

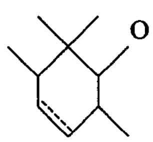

(IIe)

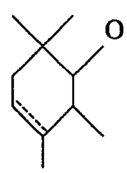

(IIf)

und

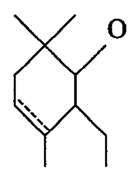

(IIg)

bedeutet, worin die gestrichelten Linien die Stellung einer Einfach- oder einer Doppelbindung bezeichnen.

3. Eine der unten angegebenen Verbindungen gemäß einem der Ansprüche 1 und 2:

1-(2,6,6-Trimethyl-cyclohex-1-yloxy)-pentan-2-ol,
1-(2,6,6-Trimethyl-cyclohex-3-en-1-yloxy)-pentan-2-ol,
1-(2,5,6,6-Tetramethyl-cyclohex-1-yloxy)-pentan-2-ol,
1-(2-Ethyl-6,6-dimethyl-cyclohex-1-yloxy)-pentan-2-ol und
1-(2,6,6-Trimethyl-cyclohex-1-yloxy)-butan-2-ol.

4. Verfahren zur Herstellung einer Verbindung gemäß Anspruch 1, dadurch gekennzeichnet, daß man eine Verbindung der Formel

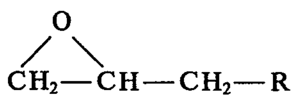

(III)

worin das Symbol R einen C-1 bis C-3 Alkylrest bedeutet, mit einer Verbindung der Formel

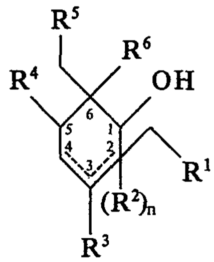

(IV)

worin die Symbole $R^1$ bis $R^6$, der Index n und die gestrichelten Linien wie in Formel (I) definiert sind, in Anwesenheit einer starken Base, umsetzt.

5. Verwendung einer Verbindung gemäß einem der Ansprüche 1 bis 3 als Parfüm- oder Aromabestandteil.

6. Parfüm- oder Aromazusammensetzung, dadurch gekennzeichnet, daß sie als aktiven Bestandteil eine Verbindung gemäß einem der Ansprüche 1 bis 3 enthält.

7. Ein parfümiertes Produkt, dadurch gekennzeichnet, daß es als geruchstragenden Bestandteil eine Verbindung gemäß einem der Ansprüche 1 bis 3 enthält.

8. Ein Nahrungsmittel, ein Getränk, ein pharmazeutisches Präparat oder ein Tabak, dadurch gekennzeichnet, daß sie als Aromabestandteil eine Verbindung gemäß einem der Ansprüche 1 bis 3 enthalten.

**Claims**

1. Compounds of formula

$$X—CH_2—CH(OH)—CH_2—R \qquad (I)$$

wherein symbol R represents an alkyl radical having 1 to 3 carbons atoms and X represents a substituted cyclohexyloxy radical of formula

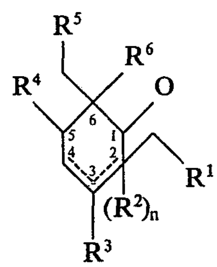

(II)

wherein index n stands for zero or 1, the dotted lines designate a single (n = 1) or a double bond in position 2 (n = 0) or 3 (n = 1) and each of symbols $R^1$ to $R^6$ represents a hydrogen atom or a methyl radical.

2. Compounds of formula (I) according to claim 1 wherein symbol X represents a substituted cyclohexyloxy radical of formula

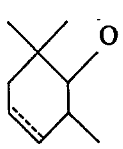

(IIa)

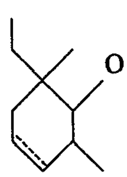

(IIb)

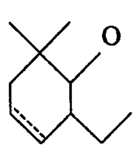

(IIc)

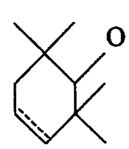

(IId)

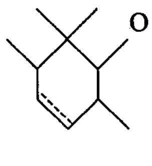 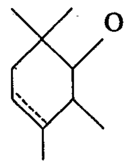 and 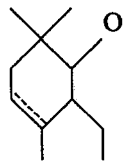

(IIe) (IIf) (IIg)

wherein the dotted lines designate the position of a single or a double bond.

3. As a compound according to one of claims 1 and 2, one of the compounds mentioned hereinbelow:

1-(2,6,6-trimethyl-cyclohex-1-yloxy)-pentan-2-ol,
1-(2,6,6-trimethyl-cyclohex-3-en-1-yloxy)-pentan-2-ol,
1-(2,5,6,6-tetramethyl-cyclohex-1-yloxy)-pentan-2-ol,
1-(2-ethyl-6,6-dimethyl-cyclohex-1-yloxy)-pentan-2-ol and
1-(2,6,6-trimethyl-cyclohex-1-yloxy)-butan-2-ol.

4. Process for the preparation of a compound according to claim 1, characterized in that a compound of formula

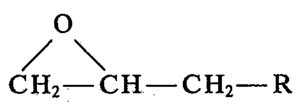

(III)

wherein symbol R represents an alkyl radical having 1 to 3 carbon atoms is reacted in the presence of a strong base with a compound of formula

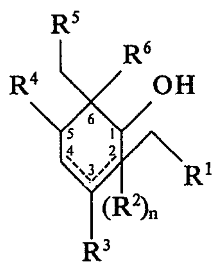

(IV)

wherein symbols $R^1$ to $R^6$, index n and the dotted lines are defined as for formula (I).

5. Utilisation of a compound according to one of claims 1 to 3 as perfuming or flavouring ingredient.

6. Perfuming or flavouring composition characterized in that it contains a compound according to one of claims 1 to 3 as organoleptically active ingredient.

7. A perfumed product characterized in that it contains a compound according to one of claims 1 to 3 as perfume ingredient.

8. A foodstuff, a beverage, a pharmaceutical preparation or a tobacco characterized in that they contain a compound according to one of claims 1 to 3 as a flavouring ingredient.